# EUROPEAN PATENT APPLICATION

(11) **EP 4 610 992 A1**
(43) Date of publication of application: **03.09.2025**
(21) Application number: 23882786.9
(22) Date of filing: 30.10.2023
(51) Int. Cl.: G16H 10/60, G16H 30/20

(54) **ANALYSIS DEVICE, ANALYSIS METHOD, ANALYSIS PROGRAM, AND RECORDING MEDIUM**

(30) Priority: 28.10.2022 JP 2022173782
(71) Applicant: Kyocera Corporation, Kyoto-shi, Kyoto 612-8501 (JP)
(72) Inventor: WATANABE, Kenichi, Kyoto-shi, Kyoto 612-8501 (JP); HONDA, Shintaro, Kyoto-shi, Kyoto 612-8501 (JP); SHIRATORI, Takaaki, Kyoto-shi, Kyoto 612-8501 (JP)
(74) Representative: TBK
(86) International application number: PCT/JP2023/039051
(87) International publication number: WO 2024/090585

(57) **Abstract**

To provide a system capable of analyzing medical data with personal information kept confidential. An analysis device includes an acquiring unit that acquires medical data that is not encrypted and second identification information obtained by encrypting first identification information of a subject from a user terminal, an analyzing unit that analyzes the acquired medical data, and a transmitter that transmits an analysis result analyzed by the analyzing unit and the second identification information to the user terminal.

## Description

### TECHNICAL FIELD

The present disclosure relates to an analysis device, an analysis method, an analysis program, and a recording medium.

### BACKGROUND OF INVENTION

For example, Patent Document 1 discloses a medical system including a medical apparatus for observing a subject and a network apparatus that can communicate with the medical apparatus.

### CITATION LIST

### PATENT LITERATURE

Patent Document 1: WO 2020/066076

### SUMMARY

In one aspect of the present disclosure, an analysis device includes an acquiring unit that acquires medical data of a subject and second identification information obtained by encrypting first identification information of the subject from a user terminal, an analyzing unit that analyzes the acquired medical data, and a transmitter that transmits an analysis result analyzed by the analyzing unit and the second identification information to the user terminal.

In one aspect of the present disclosure, an analysis method causes at least one processor to perform: acquiring medical data of a subject and second identification information obtained by encrypting first identification information of the subject from a user terminal, analyzing the acquired medical data, and transmitting an analysis result and the second identification information to the user terminal.

In each aspect of the present disclosure, the analysis device may be implemented by a computer, and in this case, the scope of the present disclosure also includes a control program of the analysis system that causes a computer to implement the analysis system by causing the computer to operate as each unit (software element) included in the analysis system, and a computer-readable non-transitory recording medium in which the control program has been recorded.

### BRIEF DESCRIPTION OF THE DRAWINGS

FIG. 1 is a block diagram illustrating a configuration of an analysis system according to a first embodiment of the present disclosure.
FIG. 2 is a schematic diagram illustrating a part of a flow of data transmitted and received between an analysis device and a user terminal.
FIG. 3 is a flowchart showing a flow of a process of an analysis method executed by a controller.
FIG. 4 is a block diagram illustrating a configuration of an analysis system according to a second embodiment of the present disclosure.
FIG. 5 is a diagram illustrating an example of a browser image.
FIG. 6 is a diagram illustrating an example of X-ray image data for requesting bone density analysis.
FIG. 7 is a schematic diagram illustrating an example of a browser image of an analysis result transmitted by a transmitter.
FIG. 8 is a diagram illustrating an example of a browser image to which derivation basis data is added in addition to analysis result data.
FIG. 9 is an enlarged schematic diagram of an image of a region illustrated in FIG. 8.
FIG. 10 is a diagram illustrating an example of a browser image displaying the risk of fracture of a patient at the present time and in the future.

### DESCRIPTION OF EMBODIMENTS

### First Embodiment

### Configuration of Analysis System 70

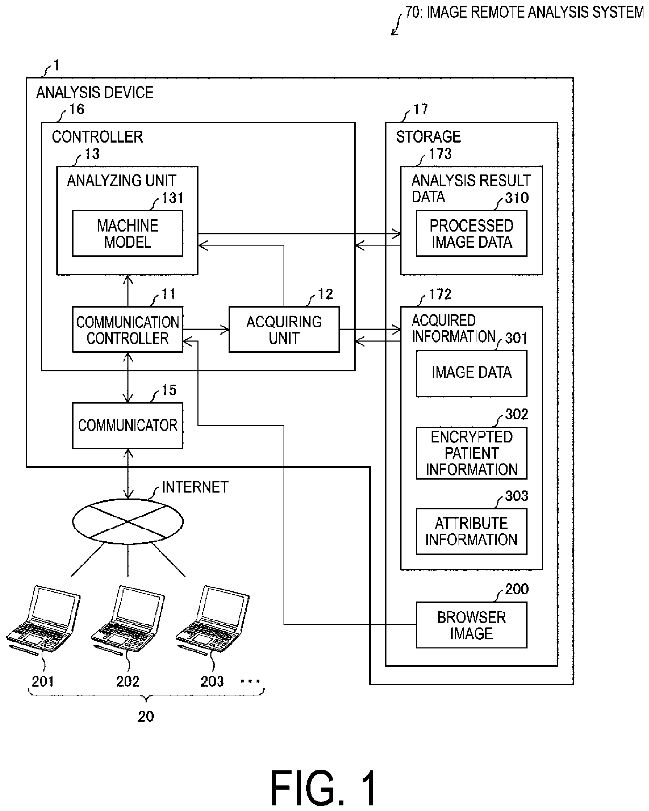

An embodiment of the present disclosure will be described in detail below with reference to accompanying drawings. FIG. 1 is a block diagram illustrating a configuration of an analysis system 70 according to a first embodiment of the present disclosure. The analysis system 70 can be used in a system for remotely analyzing images. The analysis system 70 includes, for example, an analysis device 1 that analyzes medical data and user terminals 20. In the analysis system 70, the analysis device 1 and the user terminals 20 are communicatively connected to each other via the Internet.

The analysis system 70 receives an image analysis request from the user terminals 20 via an information communication network such as the Internet. The analysis device 1 is a system that analyzes medical data to be analyzed transmitted from the user terminals 20 and returns the analysis result to the user terminals 20 via the Internet. A user may be any person who requests the analysis system 70 to analyze medical data. A user is, for example, any of medical personnel such as a doctor, a laboratory technician, or a nurse, but is not limited thereto.

In the present disclosure, medical data being image data 301 will be described as an example. However, medical data is not limited to the image data 301, and for example, blood data, blood flow data, walking data, or the like may be used as the medical data. For example, the analysis system 70 may analyze blood data and analyze motions of a patient.

There may be a plurality of user terminals 20, and for example, there may be each of user terminals 201, 202, and 203 in each medical institution. The user accesses the analysis device 1 via the Internet, for example, and requests analysis of the image data 301 from a screen displayed on a web page. If analysis requests for the same image data 301 are received from different user terminals 20, the image data 301 may be stored and analyzed in association with identification information of each of the user terminals. The image data may be in a format compliant with Digital Imaging and Communications in Medicine (DICOM).

Each user terminal 20 may be, for example, a stationary personal computer having a communication function or a mobile terminal such as a tablet terminal. Since there may be a plurality of users, the plurality of user terminals 201, 202, 203, and the like are illustrated in the drawings; however, in the following description, unless otherwise specified a target terminal will be referred to as a user terminal 20 as representative of all the terminals.

The analysis device 1 includes a controller 16 that controls each unit of the analysis device 1 in an integrated manner, a storage 17 that stores various types of data to be used by the controller 16, and a communicator 15 for communicating with other devices.

The controller 16 includes a communication controller 11 (transmitter) that controls the communicator 15, an acquiring unit 12 that acquires data transmitted from the user terminal 20 via the communication controller 11, and an analyzing unit 13 that analyzes the image data 301.

The controller 16 includes at least one processor and at least one memory. The processor may be configured by using a general-purpose processor, for example, at least one micro processing unit (MPU) or a central processing unit (CPU). The memory may include various types of memories such as a read only memory (ROM) and a random access memory (RAM). As an example, the processor realizes the functions of the controller 16 by loading various control programs recorded in the ROM of the memory to the RAM and executing the programs. The processor may also include a processor constituted by an application specific integrated circuit (ASIC), a field programmable gate array (FPGA), or a programmable logic device (PLD).

The storage 17 stores a browser image 200, acquired information 172, and analysis result data 173 (analysis result), which will be described below.

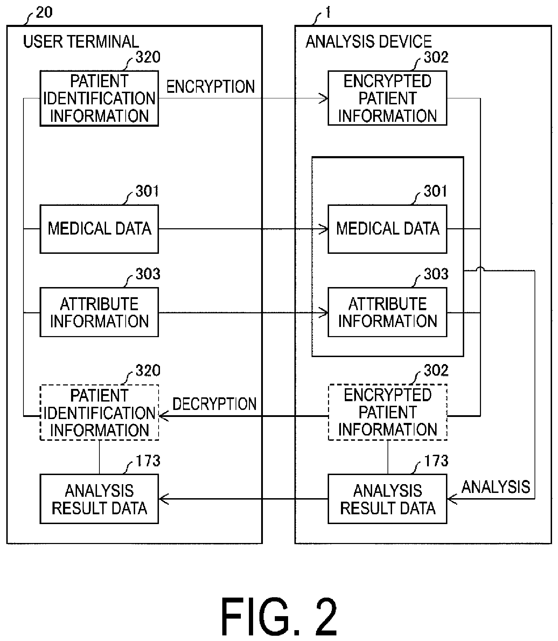

FIG. 2 is a schematic diagram illustrating a part of the flow of data transmitted and received between the analysis device 1 and a user terminal 20. When the user accesses the analysis device 1 through the Internet in order to request analysis of the image data 301, the communication controller 11 acquires a browser image 200, which is an input screen, from the storage 17 via the communicator 15 and displays the browser image 200 on a web page. The user inputs acquired information 172 including the image data 301 of the analysis target, patient identification information 320 (first identification information), and attribute information 303 to the browser image 200 and transmits the acquired information to the analysis device 1.

The user can transmit the acquired information 172 to the analysis device 1 by inputting the acquired information 172 to the browser image 200 and clicking a "transmit" button 204 to be described below. The method of input by a user is not limited to this, and for example, input may be performed by drag and drop, or input may be performed by designating a directory. The image data may be uploaded to a server while maintaining the state of a DICOM file, or only pixel values and arrangement information of the image extracted from the DICOM file or the minimum attribute information 303 embedded in the DICOM file may be transmitted. By doing this operation, anonymity can be further ensured. When the acquired information 172 is transmitted to the analysis device 1, the image data 301 and the attribute information 303 are not encrypted, but the patient identification information 320 is encrypted and transmitted as will be described in detail below. The embodiment is not limited thereto, and the image data 301 need not be encrypted, but the image data 301 may be encrypted and transmitted. A specific example of the browser image 200 will be described below.

The data type of the image data 301 may be data that can be transmitted through the Internet. Although the content of the image is not particularly limited, it may be, for example, a medical X-ray image, a microscopy photograph, a computer tomography (CT) image, a magnetic resonance imaging (MRI) image, an ultrasonic image, a PET image, or the like depending on the use of the analysis system 70. For example, when the analysis system 70 estimates bone density or the like, the type of the image data 301 is an X-ray image, a CT image, an MRI image, or the like of a bone. The estimation may include calculation. When the analysis system 70 performs pathological determination of a cell, the type of the image data 301 is data of a microscopy photograph of the cell or the like. A bone density may be represented by at least one type of bone density per unit area (g/cm²), bone density per unit volume (g/cm³), YAM (%), T-score, and Z-score. YAM (%) is an abbreviation of "Young Adult Mean" and is sometimes referred to as percentage of young adult mean. For example, a bone density may be a value expressed as bone density per unit area (g/cm²) and YAM (%). A bone density may be an index defined by a guideline or may be a unique index.

The patient identification information 320 is, for example, information indicating whose image data the image data 301 to be analyzed belongs to, and may be an ID number or information including age, address, name, image identification number, and the like. As an ID number, for example, an arbitrary combination of a plurality of alphanumeric characters can be used. An identification number of an image may be a number assigned at the time of photographing with a photographing device.

As described above, for example, the patient identification information 320 is encrypted when transmitted to the analysis device 1, and the encrypted patient information 302 (second identification information) is generated. Accordingly, the image data 301 can be transmitted and analyzed with the personal information kept confidential.

The encryption process may be performed by the user terminal 20 that transmits the patient identification information 320 or may be performed by the analysis device 1 that receives the patient identification information 320. The method of the encryption process may be a known method and is not limited.

For example, when receiving access from the user terminal 20 through the Internet, the communication controller 11 may transmit an encryption application to the user terminal 20. This encryption application is an application for encrypting the patient identification information 320 input to the browser image 200 and decrypting the encrypted patient information 302 transmitted to the user terminal 20 after analysis of the image data 301. According to this configuration, even if the user does not encrypt or decrypt the patient identification information 320 by himself/herself, the patient identification information 320 can be automatically encrypted or decrypted. The encrypted second identification information need not be displayed on the user terminal 20. The user can use the identification information without being aware of the identification information having been encrypted.

The attribute information 303 is information including information such as the name and address of the hospital in which the image data 301 was obtained, the ID number of the hospital, and the e-mail address of the hospital.

The acquiring unit 12 acquires the encrypted patient information 302 in which the image data 301, the attribute information 303, and the patient identification information 320 included in the acquired information 172 transmitted from the user terminal 20 are encrypted, and transmits the acquired encrypted patient information to the analyzing unit 13. The acquiring unit 12 stores the acquired image data 301, the encrypted patient information 302, and the attribute information 303 in the storage 17 in association with each other.

The analyzing unit 13 analyzes the image data 301 acquired from the acquiring unit 12 by using a machine model 131. The analyzing unit 13 includes the machine model 131 as illustrated in FIG. 1. The machine model 131 is a trained machine model that has been trained to output predetermined information that is an analysis result from the input image data 301. Hereinafter, analysis content based on an output of the machine model 131 is also referred to as "an analysis result derived by the machine model" or simply as "an analysis result". The data including the analysis result is referred to as analysis result data 173. The analysis result data 173 is stored in the storage 17 in association with the encrypted patient information 302 and the attribute information 303. The image data 301 may be analyzed in a format compliant with Digital Imaging and Communications in Medicine (DICOM) or may be analyzed as pixel values and arrangement information of an image extracted from a file compliant with DICOM. Alternatively, the image may be converted into another image format before being analyzed. The other image format may be a general-purpose image format such as JPG, BMP, TIF, or PNG. By doing the operation, a machine model created to target images in a general-purpose format can be used as is.

Although the machine model 131 is not limited, it may include, for example, a convolutional neural network model (NNM) for image analysis. Specific examples of the machine model 131 and the analysis result data 173 will be described in detail in a second embodiment.

The communication controller 11 associates the analysis result data 173 analyzed by the analyzing unit 13, the encrypted patient information 302, and the attribute information 303 with each other and transmits the information to the user terminal 20 via the communicator 15.

When the user terminal 20 receives the analysis result data 173, the encrypted patient information 302, and the attribute information 303, the encrypted patient information 302 is decrypted to generate the patient identification information 320. At this time, when the analysis device 1 has transmitted the encryption application to the user terminal 20, the encryption application automatically decrypts the encrypted patient information 302, and thus the user does not need to perform decryption. Here, decryption refers to, for example, restoring the original patient identification information 320 from the encrypted patient information 302.

In this way, the user terminal 20 can receive the decrypted patient identification information 320, the analysis result data 173, and the attribute information 303. Accordingly, the user terminal 20 can store the analysis result data 173 in association with the patient identification information 320.

In FIG. 1, the analysis device 1 is illustrated as being arranged together in one housing. However, the analysis device 1 does not need to be arranged together in one housing. For example, some of the components included in the analysis device 1 may be arranged as separate devices. Some or all of the components included in the analysis device 1 may be arranged on a cloud.

According to the analysis system 70 having the above-described configuration, the image data 301 requested to be analyzed by the user can be analyzed with the personal information kept confidential. Thus, even when an analysis request is received from an unspecified number of users, the image data 301 of each user can be analyzed while protecting the personal information.

The image data 301 may be, for example, medical image data obtained by imaging a subject with an endoscope. More specifically, the image data 301 may include medical image data obtained by imaging a site including at least one of the subject's nasal cavity, esophagus, stomach, duodenum, rectum, large intestine, small intestine, anus, and colon with an endoscope. By inputting the medical image data obtained by imaging the site to the machine model 131, for example, an analysis result indicating a site of interest including at least one of inflammation, polyp, or cancer may be output. In such a case, as the machine model 131, for example, a model trained based on a first training image including an image with the site of interest, first teacher data indicating the presence of the site of interest, a second training image including an image without the site of interest, and second teacher data indicating the absence of the site of interest can be used. The first teacher data may include information indicating the inflammation degree (degree of inflammation) or the malignancy degree (degree of malignancy) of the site of interest. The analysis result may be, for example, a display surrounding the site of interest, a display indicating the site of interest, or a display in which a color is superimposed on the site of interest. In addition to the analysis result, derived basis information indicating the basis for deriving the analysis result may be displayed.

Alternatively, the image data 301 may be, for example, image data in which an image of the subject's eyes, skin, or the like has been captured with a digital camera. The image data 301 obtained by capturing images of these sites may be input to the machine model 131 to output, for example, an analysis result clearly indicating a sign of interest. The sign of interest may include, for example, signs indicating diseases including at least one of glaucoma, cataracts, age-related macular degeneration, conjunctivitis, styes, retinopathy, or blepharitis. Alternatively, the sign of interest may include, in the case of skin, indications including, for example, skin cancer, urticaria, atopic dermatitis, herpes, and the like. The analysis result may be a display surrounding these signs of interest, a display indicating the signs of interest, a display in which a color is superimposed on the signs of interest, or may display the disease name. As the machine model 131, for example, a model trained based on the first training image including an image with the site of interest, first teacher data indicating the presence of the signs of interest, a second training image including an image without the signs of interest, and second teacher data indicating the absence of the signs of interest can be used. In addition to the analysis result, derived basis information indicating the basis for deriving the analysis result may be displayed.

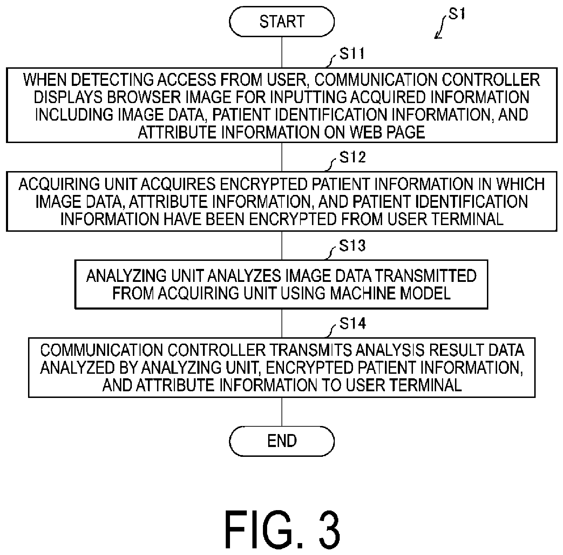

A flow of an analysis method S1 according to the present embodiment will be described with reference to drawings. FIG. 3 is a flowchart showing the flow of the process of the analysis method S1 executed by the controller 16. The analysis method S1 includes steps S11 to S14 as illustrated.

When the user accesses the analysis device 1, the communication controller 11 displays the browser image 200, which is an input screen for inputting the acquired information 172 including the image data 301 to be analyzed, the patient identification information 320, and the attribute information 303 on the web page via the communicator 15 (S11).

The acquiring unit 12 acquires the encrypted patient information 302 in which the image data 301, the attribute information 303, and the patient identification information 320 included in the acquired information 172 input in the browser image 200 and transmitted from the user terminal 20 are encrypted (S11).

The analyzing unit 13 analyzes the image data 301 transmitted from the acquiring unit 12 by using the machine model 131 (S13).

The communication controller 11 transmits the analysis result data 173 analyzed by the analyzing unit 13, the encrypted patient information 302, and the attribute information 303 to the user terminal 20 via the communicator 15 (S14).

According to the analysis method S1 described above, the image data 301 requested to be analyzed by the user can be analyzed with the personal information kept confidential. Thus, even when an analysis request is received from an unspecified number of users, the image data can be analyzed while protecting the personal information.

### Second Embodiment

Another embodiment of the present disclosure will be described below. For convenience of description, members having the same functions as those of the members described in the above-described first embodiment are denoted by the same reference signs, and description thereof is not repeated. In the present embodiment, an example in which an analysis system 70A analyzes X-ray images of bones and outputs bone density, relative comparison of bone density, likelihood of fracture (%), and the like will be described.

### Configuration of Analysis System 70A

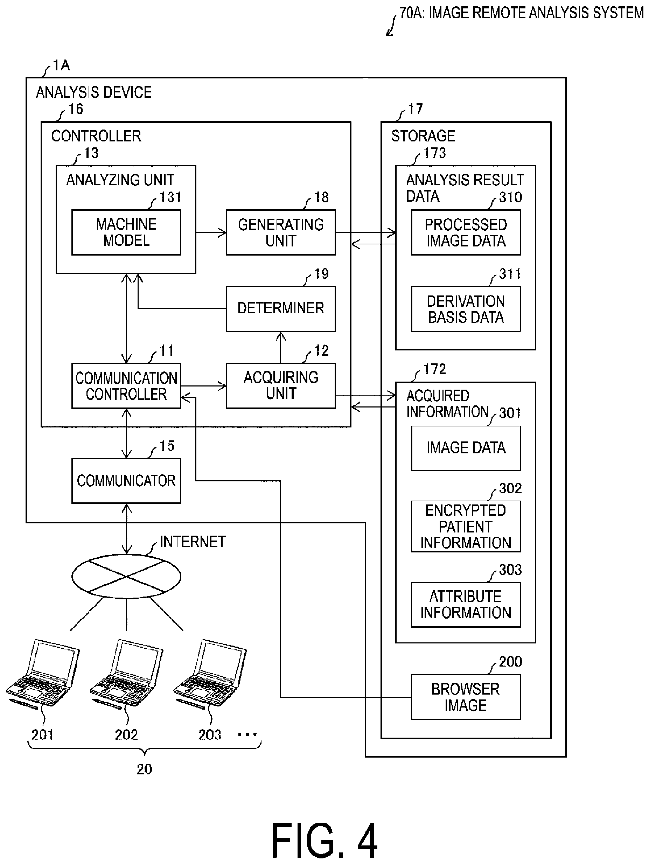

FIG. 4 is a block diagram illustrating a configuration of the analysis system 70A according to a second embodiment of the present disclosure. As illustrated in the drawing, a controller 16 of an analysis device 1A includes a communication controller 11, an acquiring unit 12, an analyzing unit 13, a generating unit 18, and a determiner 19. Among them, the communication controller 11, the acquiring unit 12, and the analyzing unit 13 have the same functions as those of the respective units described in the first embodiment, and thus description thereof is omitted here.

A storage 17 stores image data 301 (medical data) acquired by the acquiring unit 12, encrypted patient information 302, and attribute information 303 in association with each other.

The generating unit 18 generates information (hereinafter referred to as "derivation basis data 311") related to the derived basis of an analysis result analyzed by the analyzing unit 13. The derivation basis data 311 means at least one basis for why the machine model 131 derived such analysis results. For example, the derivation basis data 311 may be information mainly indicating which part of an image is used as a basis to derive the analysis result. The derivation basis data 311 is associated with at least the encrypted patient information 302 and is stored in the storage 17. A specific example of the function of the generating unit 18 will be described below.

### Determiner 19

The determiner 19 determines whether the encrypted patient information 302 acquired by the acquiring unit 12 is information obtained by actually encrypting the patient identification information 320. When it is determined that the encrypted patient information 302 was not actually encrypted, the acquiring unit 12 may delete the acquired encrypted patient information 302. By providing such a determiner 19, if the encrypted patient information 302 transmitted from the user terminal 20 is not information obtained by encrypting the patient identification information 320, the acquired image data 301 is deleted together with the encrypted patient information 302 and the attribute information 303 without being analyzed. Thus, the acquired information 172 including the image data 301 is not stored inside the analysis device 1A. Whether data has been encrypted may be determined from, for example, the extension of the encrypted data.

When it is determined that the encrypted patient information 302 has not been actually encrypted, the controller 16 may transmit a message requesting that the patient identification information 320 be encrypted and transmitted again to the user terminal 20 via the communication controller 11.

Although a configuration in which the analysis device 1A includes the determiner 19 is described in the present disclosure, the configuration is not limited thereto. For example, in the analysis system 70A, the determiner 19 may be included in at least one of the analysis device 1A or a hospital-side server that mediates transmission of the encrypted patient information 302 from the user terminal 20 to the analysis device 1A. In one example, both the analysis device 1A and the hospital-side server may include the determiner 19. The user terminal 20 that is the transmission source of the encrypted patient information 302 may be configured to include the determiner 19.

### Browser Image 200

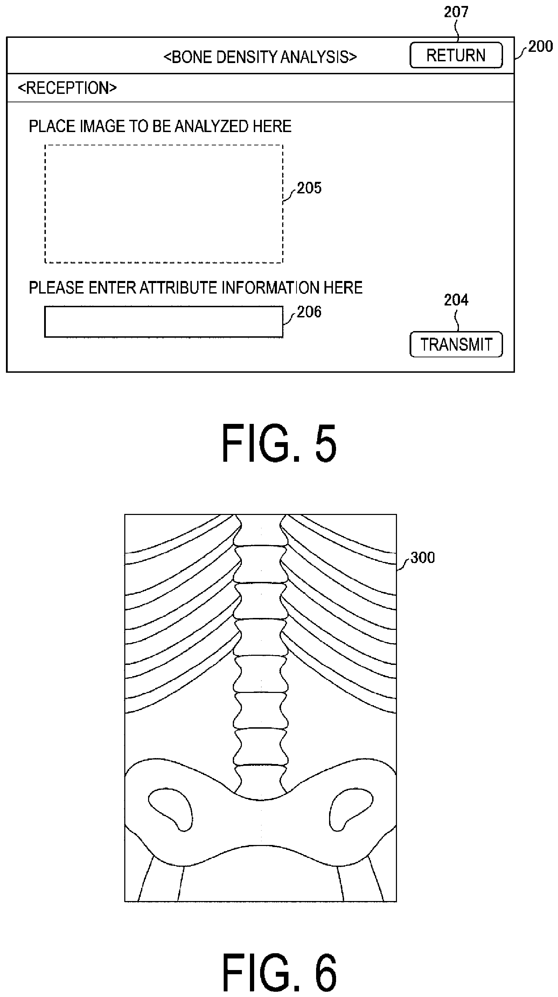

FIG. 5 is a diagram illustrating an example of a browser image 200 displayed on a web page for inputting image data 301 and the like when a user accesses the analysis device 1 through the Internet.

As illustrated in the drawing, the text "Bone Density Analysis" indicating the target of image analysis is displayed at the top of the browser image 200. At the upper left part of the browser image 200, the text "Reception" indicating that the screen is for accepting input is displayed. At a lower part thereof, the text "place an image to be analyzed here" for prompting input of image data to be analyzed and a frame 205 indicating an area (basis area) where the image data is pasted (dragged and dropped) are displayed. Furthermore, at a lower part thereof, the text "Please input attribute information here" for prompting input of the patient identification information 320, the attribute information 303, and the like, and a box 206 for input are displayed. The "transmit" button 204 for prompting transmission is displayed at the lower right part of the browser image 200, and the "return" button 207 for returning to the initial screen of the website is displayed at the upper right part.

FIG. 6 is a diagram illustrating X-ray image data 300 for requesting bone density analysis as an example of the image data 301. The X-ray image data 300 may be a waist X-ray image, a chest X-ray image, or the like, or may be an X-ray image captured by a dual energy X-ray absorptiometry (DXA) apparatus or the like. In a DXA apparatus that measures bone density using the DXA method, when the bone density of the lumbar vertebrae is measured, X-rays are emitted to the lumbar vertebrae of a subject from the front of the lumbar vertebrae. In the DXA apparatus, when the bone density of the proximal femur is measured, X-rays are emitted to the proximal femur of the subject from the front of the proximal femur. Here, the "front of the lumbar vertebrae" and the "front of the proximal femur" are intended to be directions correctly facing capturing sites such as the lumbar vertebrae and the proximal femur, and may be the ventral side of the subject's body or the back side of the subject. In the micro densitometry (MD) method, x-rays are emitted to hands. An ultrasonic method is a method of measuring bone density by applying ultrasonic waves to a bone such as the lumbar vertebrae, femur, heel, or shank. The image data need not be an X-ray image but may be an image including information of bones. For example, it can be estimated from a magnetic resonance imaging (MRI) image, a computed tomography (CT) image, a PET image, an ultrasonic image, or the like.

Although a case in which a target whose bone state is estimated is a human (that is, a "subject") is described as an example in the present disclosure, the target is not limited to a human. The subject whose bone state is estimated may be a mammal other than a human, such as any of Equidae, Felidae, Canidae, Bovidae or Suidae. The present disclosure also includes embodiments in which the term "subject" is reworded as "animal" when the embodiments are applicable to any of these animals.

In the present embodiment, when the user accesses the analysis device 1A through the Internet, the communication controller 11 may display the browser image 200 on the web page and transmit an encryption application via the communicator 15. When the user inputs the image data 301 to be analyzed, the patient identification information 320, and the attribute information 303 and clicks the "transmit" button 204, the patient identification information 320 is encrypted, and the encrypted patient information 302 is transmitted to the analysis device 1A together with the image data 301 and the attribute information 303. Therefore, the user does not need to perform an encryption process for the patient identification information 320. The encryption process need not be performed when the user clicks the "transmit" button 204, but may be performed after the user clicks the "transmit" button.

The method of the encryption process may be a known method and is not limited. For example, an encryption method in which a public key and a private key are combined may be used. The encryption may be a method by which even the operator of the analysis system 70A is not able to decrypt the information. Accordingly, even when the user requests the operator of the analysis system 70A to perform image analysis, a risk of the combination of the image data 301 and the patient identification information 320 being leaked to the operator can be reduced.

The acquiring unit 12 acquires the X-ray image data 300, the encrypted patient information 302, and the attribute information 303 transmitted from the user terminal 20, and transmits the information to the analyzing unit 13.

The analyzing unit 13 inputs the X-ray image data 300 transmitted from the acquiring unit 12 to the machine model 131, processes output data from the machine model 131 as necessary, and generates analysis result data 173. The generated analysis result data 173 is associated with at least the encrypted patient information 302 and the attribute information 303, and stored in the storage 17.

The communication controller 11 acquires the analysis result data 173 associated with the encrypted patient information 302 and the attribute information 303 from the storage 17 and transmits the acquired data to the user terminal 20 via the communicator 15.

When the user terminal 20 receives the analysis result data 173, the encrypted patient information 302 is automatically decrypted to generate the patient identification information 320. The encrypted patient information 302 may be decrypted when the analysis result data 173 is transmitted, or may be decrypted by the user himself/herself.

Accordingly, the user terminal 20 can display the analysis result data 173 on the screen together with the patient identification information 320 including the name, the identification number, or the like of the patient.

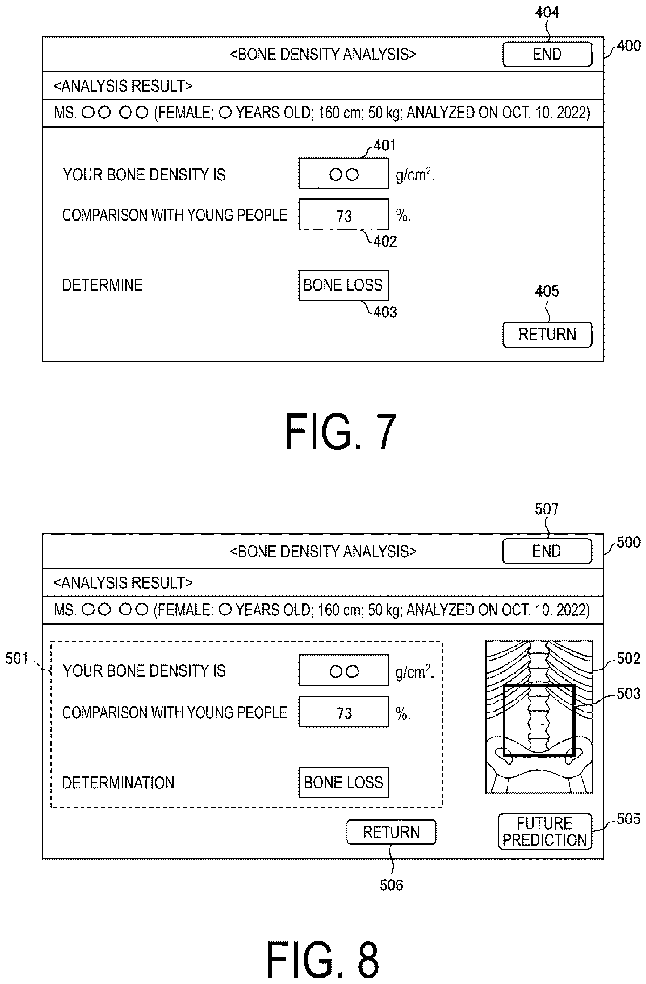

FIG. 7 is a diagram illustrating an example of a browser image 400 displayed on the screen of the user terminal 20 that has received the analysis result data 173. The text "Bone Density Analysis" indicating the content of image analysis is displayed at the top of the browser image 400. In the upper left part of the browser image 400, the text "Analysis Result" indicating that the screen is displaying the analysis result is displayed. Patient's information may be displayed therebelow. The text "Your bone density is □/cm²" is displayed therebelow. A numerical value of the estimated bone density is displayed in a box 401. Further, the text "□% of the bone density of young people" may be displayed below it. In a box 402, the average bone density in young adults, that is, the Young Adult Mean (YAM) is displayed. Further, the text "determination" may be displayed below it, and text such as "bone loss" may be displayed in a box 403. For example, if the percentage to YAM is less than 80%, the patient is determined to have "bone loss", and if the percentage is 70% or less, the patient is determined to maybe have "osteoporosis". The browser image 400 may include an "end" button 404 and a "return" button 405. For example, the "return" button 405 may be to return to the previous screen, may be to return to the home screen, or may be to return to a predetermined screen.

### Generating Unit 18

The generating unit 18 will be described in detail. In the present embodiment, the generating unit 18 generates derivation basis data 311 of an analysis result obtained from analysis by the analyzing unit 13. The communication controller 11 may transmit the derivation basis data 311 generated by the generating unit 18 to the user terminal 20.

Although the analysis result data 173 is based on the output from the machine model 131, the output from the machine model 131 does not include the process of analysis. Thus, it is general that the reliability of the output cannot be determined only by looking at the output from the machine model 131. Therefore, the transmission of the analysis result data 173 including the derivation basis data 311 to the user terminal 20 helps enhancement of the reliability of the user in the analysis result.

For example, the derivation basis data 311 may be the processed image data 310 (basis image data) obtained by adding new information to the image data 301 input to the machine model 131. For example, the new information may be information indicating a region serving as a main basis for deriving an analysis result in the image data 301. That is, the processed image may be an image obtained by adding information indicating a region serving as a main basis to the input image data. The information indicating a region is, for example, information indicating the range of the region, such as coloring or framing. In image analysis, there are many cases in which a certain region of an image serves as a main basis for estimation, and thus, the user can confirm the region serving as the basis for estimation based on information indicating such a region.

The processed image data 310 does not need to include all pieces of information of the image data 301 input by the user. For example, the processed image data 310 may be image data obtained by trimming the input image data 301, or may be an image having a resolution lower than that of the input image data 301. In this way, the amount of data to be transmitted and received can be reduced.

FIG. 8 is a diagram illustrating an example of a browser image 500 in which the derivation basis data 311 is transmitted to the user terminal 20 in addition to the analysis result data 173. In the browser image 500, the derivation basis data 311 (processed image data 502 including an area 503) is added to the analysis result data 173 illustrated in FIG. 7.

Specifically, the browser image 500 displays, together with the analysis result 501, processed image data 502 obtained by adding a rectangular area 503 to the input image data (the X-ray image data 300 in FIG. 6). As illustrated in FIG. 8, in the present embodiment, the analysis result 501 derived by the machine model 131 that analyzes the image data and the processed image data 502 including the derivation basis data 311 are displayed on one screen. In the browser image 500, a "return" button 506, an "end" button 507, and a "future prediction" button 505 may be displayed. The role of the "future prediction" button 505 will be described below.

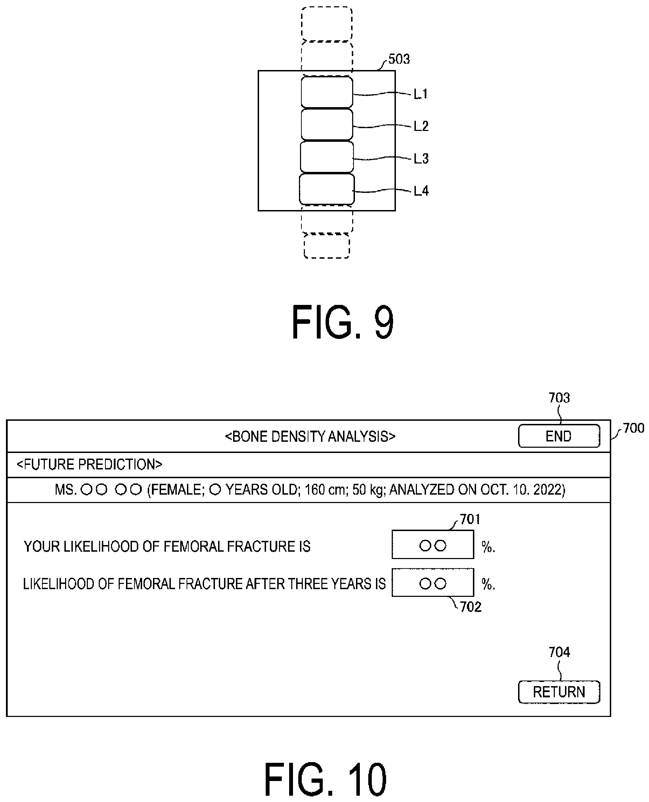

FIG. 9 is an enlarged schematic view of the image of the area 503 of FIG. 8. The area 503 includes four lumbar vertebrae indicated by L1 to L4. This indicates that the lumbar vertebrae L1 to L4 are the areas serving as the basis of the analysis result. Actually, it is known that the bone density of lumbar vertebrae L1 to L4 is related to the mean value of the bone density of the whole body. That is, the framed area 503 indicates that the analysis result of the machine model 131 is derived based on the bone densities of the lumbar vertebrae L1 to L4.

The area serving as the basis of the analysis result may include a segmented area. Segmentation is dividing an image into several areas. Segmentation is performed to reduce the amount of the analysis process of the machine model 131. That is, the machine model 131 may analyze only the segmented areas. The segmented areas may be set to an arbitrary range. Each segmented area may be, for example, a rectangle, a square, or a circle. When the segmented area is a square, the amount of analysis process of the machine model 131 can be reduced. When the X-ray image of the lumbar vertebra is analyzed, for example, the range including the lumbar vertebrae L1 to L4 is segmented. Therefore, the size of the segmented areas may change depending on the size of the lumbar vertebrae L1 to L4 in the image. In the arrangement direction of the lumbar vertebrae L1 to L4, for example, the segmented areas may be set to be slightly larger than the lumbar vertebrae L1 to L4, or may be set to overlap the sides of the lumbar vertebrae L1 to L4. For example, the segmented areas may always have a predetermined size, or may be set to have a size according to the medical image. For example, the segmented areas may be set by specifying the positions of the lumbar vertebrae L1 to 4, setting the lengths of the lumbar vertebrae L1 to L4 in the arrangement direction, and then setting the lengths of the lumbar vertebrae L1 to L4 in the vertical direction. The segmentation may be performed by the machine model 131. The machine model 131 may have learned an annotated image of the analysis area in order to perform segmentation.

The generating unit 18 may generate a heat map of the area serving as the basis of the analysis result. In this case, for example, the outer edge of the heat map indicates the segmented areas. A heat map is a method of representing the magnitude of bone density by the concentration of an arbitrary color. For example, the generating unit 18 may generate a heat map indicating the degree of focus. A heat map indicating numerical values of bone density may be generated. The generating unit 18 may generate a heat map indicating likelihood (probability) of fracture. Images used for the heat map may be still images or moving images. By representing a heat map with a moving image, for example, by fading various heat maps in order, the relationship between heat maps can be visually recognized with ease. When an analysis result is a heat map of bone density including areas other than the segmented areas, a part of the segmented areas may be surrounded by a frame.

The generating unit 18 may acquire, from the analyzing unit 13, information on the area serving as the basis of the analysis result. Specifically, the generating unit 18 may acquire the area serving as the basis of the analysis result from the analyzing unit 13 and generate information indicating the area (a frame line surrounding the area 503 or the like). The generating unit 18 may acquire the degree of focus data, the bone density data, or the information indicating the likelihood of fracture in the area from the analyzing unit 13 and generate a heat map. The generating unit 18 may generate any two or more of a heat map indicating the degree of focus data, a heat map indicating the bone density data, and a heat map indicating the likelihood of fracture. When the heat map indicating the degree of focus data, the heat map indicating the bone density data, and the heat map indicating the likelihood of fracture are displayed in an overlapping manner, the generating unit 18 may make the color of the heat map indicating the degree of focus data, the color of the heat map indicating the bone density data, and the color of the heat map indicating the likelihood of fracture different from each other.

The machine model 131 analyzes the X-ray image data 300 by NNM, for example. In NNM, processing in which an image is once divided into small areas, each of which is quantified, and a plurality of areas are subjected to pooling processing, integrated into a larger region, and quantified again is repeated. Therefore, the machine model 131 may extract an area having a numerical value (for example, a relatively large numerical value) that affects the processing result as an area serving as a basis.

In the example illustrated in FIG. 8, the processed image data 502 displayed by superimposing the area 503 serving as the basis of the analysis result on the X-ray image data 300 serving as the analysis target has been described. However, the processed image data is not limited thereto. For example, the analysis device 1 may transmit position information (coordinates or the like) of the area serving as a basis of the analysis result in the image to be analyzed to the user terminal 20 and cause the user terminal 20 to display the processed image data in which the area is displayed in the image to be analyzed.

The screen shown in FIG. 8 can be used when a doctor explains the analysis result to the patient. That is, it is possible not only to explain the analysis result to the patient but also to explain the area of the image data that served as the basis for the analysis result. Therefore, not only can the reliability of the doctor with respect to the analysis result be improved but also an effect that the patient is easily satisfied with the analysis result is obtained.

In the example illustrated in FIG. 8, the analysis result 501 and the processed image data 502 including the derivation basis data 311 are displayed on one screen. However, "one screen" may not be simultaneously displayed on the screen. For example, the screen may be scrolled up and down or left and right for display. That is, a range in which the screen is displayed by scrolling the screen up and down or left and right is referred to as "one screen".

FIG. 10 is a schematic diagram illustrating an example of a browser image 700 that displays the likelihood of fracture of the patient at the current time point estimated from the image data and the likelihood of fracture of the patient after three years, which are analyzed by the analyzing unit 13. The future time is not limited to three years later and may be any time (for example, X years later). This is displayed by clicking the "future prediction" button 505 at the lower right part of the browser image 500 shown in FIG. 8.

The text "Bone Density Analysis" indicating the target of image analysis is displayed at the top of the browser image 700. In the upper left part of the browser image 700, the text "future prediction" indicating that the screen displays future prediction is displayed. Patient's information may be displayed therebelow. At the lower part, the text "Your likelihood of femoral fracture is □%" is displayed. In a box 701, the numerical value of the likelihood of fracture estimated at the present time is displayed. The text "The likelihood of femoral fracture after three years is □%" may be displayed below it. A box 702 displays the numerical value of the predicted likelihood of fracture after three years. Information on the estimated basis or the predicted basis may be displayed together with the image.

Such a screen can be used when a doctor explains the likelihood of fracture at the current time point and in the future. When information on the estimated basis or the predicted basis is also displayed, an effect of increasing persuasion to the patient can be obtained.

### Machine Model 131

The machine model 131 will be described. The machine model 131 is a model for estimating a state of a bone, input image data is an image including a bone, and an estimation result regarding the state of the bone is output as an analysis result. For example, the machine model 131 is a trained model that has been trained to output an estimation result or a calculation result regarding a state of a bone, such as bone density, relative comparison of bone densities, presence or absence of a fracture, or likelihood of a fracture, from an X-ray image of the bone. The bone density may be a calculated bone density of a bone site included in the image data, or may be an average bone density of the whole body estimated from the image data. As a method of calculating a bone density from an image, a known method can be used. The relative comparison of bone densities is the ratio of estimated bone density with respect to YAM. The presence or absence of a fracture is information indicating whether there is a fracture in input image data. The probability of a fracture is a probability that a bone at a particular site (for example, the femoral neck) fractures. An estimation result regarding a state of a bone may be an estimation result at the time when an image was captured or may be prediction at the time after a predetermined period elapsed from the aforementioned time.

The machine model 131 may output, as the estimation result, at least one of the bone density estimated at the time when the image data was captured, the bone density predicted at the time when a predetermined period elapses from the time when the image data was captured, the fracture site estimated at the time when the image data was captured and the likelihood thereof, and the fracture site predicted at the time when a predetermined period elapses from the time when the image data was captured and the likelihood thereof.

### Training Method for Machine Model 131

The method of training the machine model 131 will be described. Training of the machine model 131, for example, training of estimating a bone density may be performed using an X-ray image of a bone whose bone density has been specified as teacher data. Training of estimating likelihood of a fracture may be performed by using an X-ray image of a bone and data indicating whether the patient suffered fracture within a predetermined period thereafter as teacher data. The relative comparison of bone densities does not have to be a subject for training and is obtained by dividing the estimated bone density by the YAM. Training of future prediction may be performed using an X-ray image of a bone whose bone density has been specified and data indicating the bone density of the patient after a predetermined period of time thereafter or whether the patient has suffered fracture as teacher data. The machine model 131 capable of performing more accurate estimation or prediction by including, in the teacher data, lifestyles such as an exercise amount, types of food for meals, smoking, and drinking of the patient serving as the teacher data.

In the second embodiment, a machine model trained to estimate a state of a bone such as a bone density has been described as an example of the machine model 131. In this case, the input image data is an X-ray image of the bone, and the output is an estimation result regarding the state of the bone. However, the machine model 131 is not limited to such a model. For example, the machine model 131 may be a cytopathology analysis model. In this case, the input image data may be a microscopic image of a cell, and the output may be the presence or absence of a pathological mutation of the cell. Alternatively, the image data may be an X-ray image, a CT image, a mammographic image, or the like, and the output may be the presence or absence of a cancer.

According to the configuration of the analysis system 70A of the second embodiment described above, the derivation basis data 311 can be provided to the user together with the analysis result data 173. For this reason, in addition to the effect of the analysis system 70 according to the first embodiment, an effect of improving the user's confidence in the analysis result can be exhibited. An effect of helping the patient easily understand the analysis result when explanation is provided to the patient can be obtained.

The flow of an analysis method S2 performed by the controller 16 according to the second embodiment will be described. The analysis method S2 includes steps S21 to S24. Steps S21 to S23 are the same as steps S11 to S13 described in the analysis method S1 described in the first embodiment.

In step S24, the communication controller 11 transmits the analysis result data 173 analyzed by the analyzing unit 13, the derivation basis data 311, and the encrypted patient information 302 to the user terminal 20 (not illustrated).

According to the analysis method S2 described above, the derivation basis data 311 can be provided to the user together with the analysis result data 173. For this reason, in addition to the effect of the analysis method S1 according to the first embodiment, the effect of improving the user's confidence in the analysis result can be exhibited. The effect of helping the patient easily understand the analysis result when explanation is provided to the patient can be obtained.

### Implementation Example by Software

The functions of the analysis systems 70 and 70A (hereinafter, referred to as "systems") can be implemented by a program for causing a computer to function as the systems, the program for causing a computer to function as each unit of the systems.

In this case, each of the systems includes a computer having at least one control device (e.g., processor) and at least one storage device (e.g., memory) as hardware for executing the program. By executing the program by the control device and the storage device, the functions described in the embodiment are implemented.

The program may be recorded on one or a plurality of computer-readable non-transitory recording media. This recording media may be or need not be included in the device. In the latter case, the program may be supplied to the apparatus via any wired or wireless transmission medium.

Some or all of the functions of each of the above-described units can be implemented by logic circuits. For example, an integrated circuit in which logic circuits functioning as each of the above-described units is also included in the scope of the present disclosure. In addition to this, for example, a quantum computer can implement the functions of each of the above-described units.

The invention according to the present disclosure has been described above based on the drawings and examples. However, the invention according to the present disclosure is not limited to each embodiment described above. That is, the embodiments of the invention according to the present disclosure can be modified in various ways within the scope illustrated in the present disclosure, and embodiments obtained by appropriately combining the technical means disclosed in different embodiments are also included in the technical scope of the invention according to the present disclosure. In other words, note that a person skilled in the art can easily make different variations or modifications based on the present disclosure. Note that these variations or modifications are within the scope of the present disclosure.

### Conclusion

### First Aspect

An analysis device of a first aspect of the present disclosure includes an acquiring unit configured to acquire medical data of a subject and second identification information obtained by encrypting first identification information of the subject from a user terminal; an analyzing unit configured to analyze the acquired medical data; and a transmitter configured to transmit an analysis result analyzed by the analyzing unit and the second identification information to the user terminal.

### Second Aspect

An analysis device of a second aspect of the present disclosure according to the first aspect may transmit the analysis result and the second identification information to the user terminal that decrypts the second identification information.

### Third Aspect

In an analysis device of a third aspect of the present disclosure according to the first or second aspect, the user terminal may include an application that performs decryption, and the application may have a function of performing the encryption.

### Fourth Aspect

An analysis device of a fourth aspect of the present disclosure according to the third aspect may transmit an application main body that performs the decryption to the user terminal.

### Fifth Aspect

An analysis device of a fifth aspect of the present disclosure according to any one of the first to fourth aspects may not have the second identification information displayed on the user terminal.

### Sixth Aspect

An analysis device of a sixth aspect of the present disclosure according to any one of the first to fifth aspects may further include a determiner configured to determine whether the acquired second identification information has been obtained by encrypting the first identification information in a predetermined method, in which the acquiring unit may delete the acquired second identification information when it is determined that the second identification information has not been obtained by encrypting the first identification information in the predetermined method.

### Seventh Aspect

In an analysis device of a seventh aspect of the present disclosure according to any one of the first to sixth aspects, the medical data may be image data.

### Eighth Aspect

In an analysis device of an eighth aspect of the present disclosure according to any one of the first to seventh aspects, the medical data may be image data in which an image of a bone of the subject has been captured, and the analyzing unit may output an estimation result regarding a state of the bone of the subject as the analysis result.

### Ninth Aspect

An analysis device of a ninth aspect of the present disclosure according to in any one of the first to eighth aspects may further include a generating unit configured to generate basis information regarding a derivation basis of the analysis result, in which the transmitter may include the basis information in the analysis result and transmit the analysis result to the user terminal.

### Tenth Aspect

In an analysis device of a tenth aspect of the present disclosure according to the ninth aspect, the medical data may be image data, and the basis information may be basis image data in which information indicating a basis area serving as a basis for deriving the analysis result in the medical data has been added to the medical data.

### Eleventh Aspect

In an analysis device of an eleventh aspect of the present disclosure according to the tenth aspect, the basis image data may be image data in which the basis area is shown on the image data.

### Twelfth Aspect

In an analysis device of a twelfth aspect of the present disclosure according to the eighth aspect, the analyzing unit may output, as an estimation result, at least one of a bone density of the bone of the subject at a time when the image data was captured, a bone density of the bone of the subject at a time when a predetermined period elapsed from the time when the medical image data was captured, a site where a fracture is estimated to occur at the time when the medical image data was captured and likelihood of the fracture occurring, and a site where a fracture is estimated to occur from the time when the medical image data was captured until a predetermined period elapses and likelihood of the fracture occurring.

### Thirteenth Aspect

An analysis method according to a thirteenth aspect of the present disclosure includes causing at least one processor to perform: acquiring medical data of a subject and second identification information obtained by encrypting first identification information of the subject from a user terminal; analyzing the acquired medical data; and transmitting an analysis result and the second identification information to the user terminal.

### Fourteenth Aspect

An analysis program according to a fourteenth aspect of the present disclosure is an analysis program for causing a computer to function as the analysis device described in at least one of the first to twelfth aspects, and a computer program for causing a computer to function as the acquiring unit, the analyzing unit, and the transmitter.

### Fifteenth Aspect

A recording medium according to a fifteenth aspect of the present disclosure is a computer readable non-transitory recording medium in which the analysis program described in the fourteenth aspect has been recorded.

### REFERENCE SIGNS

1, 1A Analysis device
11 Communication controller (transmitter)
12 Acquiring unit
13 Analyzing unit
18 Generating unit
19 Determiner
20 User terminal
131 Machine model
173 Analysis result data (analysis result)
301 Image data (medical image data)
302 Encrypted patient information (second identification information)
310 Processed image data (basis image data)
311 Derivation basis data (information on derivation basis of analysis result)
320 Patient identification information (first identification information)
503 Area (basis area)

## Claims

1. An analysis device comprising:
an acquiring unit configured to acquire medical data of a subject and second identification information obtained by encrypting first identification information of the subject from a user terminal;
an analyzing unit configured to analyze the acquired medical data; and
a transmitter configured to transmit an analysis result analyzed by the analyzing unit and the second identification information to the user terminal.

2. The analysis device according to claim 1, wherein
the analysis result and the second identification information are transmitted to the user terminal that decrypts the second identification information.

3. The analysis device according to claim 1 or 2, wherein
the user terminal comprises an application that performs decryption, and
the application comprises a function of performing the encryption.

4. The analysis device according to claim 3, wherein
an application main body that performs the decryption is transmitted to the user terminal.

5. The analysis device according to any one of claims 1 to 4,
wherein
the second identification information is not displayed on the user terminal.

6. The analysis device according to any one of claims 1 to 5, further comprising:
a determiner configured to determine whether the acquired second identification information has been obtained by encrypting the first identification information in a predetermined method, wherein
the acquiring unit deletes the acquired second identification information when it is determined that the second identification information has not been obtained by encrypting the first identification information in the predetermined method.

7. The analysis device according to any one of claims 1 to 6, wherein
the medical data is image data.

8. The analysis device according to any one of claims 1 to 7, wherein
the medical data is image data in which an image of a bone of the subject has been captured, and
the analyzing unit outputs an estimation result regarding a state of the bone of the subject as the analysis result.

9. The analysis device according to any one of claims 1 to 8, further comprising:
a generating unit configured to generate basis information regarding a derivation basis of the analysis result, wherein
the transmitter comprises the basis information in the analysis result and transmits the analysis result to the user terminal.

10. The analysis device according to claim 9, wherein
the medical data is image data, and
the basis information is basis image data in which information indicating a basis area serving as a basis for deriving the analysis result in the image data has been added to the image data.

11. The analysis device according to claim 10, wherein
the basis image data is image data in which the basis area is shown on the image data.

12. The analysis device according to claim 8, wherein
the analyzing unit outputs, as an estimation result, at least one of a bone density of a bone of the subject at a time when the image data was captured, a bone density of the bone of the subject at a time when a predetermined period elapsed from the time when the image data was captured, a site where a fracture is estimated to occur at the time when the image data was captured and likelihood of the fracture occurring, and an site where a fracture is estimated to occur from the time when the image data was captured until a predetermined period elapses and likelihood of the fracture occurring.

13. An analysis method causing at least one processor to perform:
acquiring medical data of a subject and second identification information obtained by encrypting first identification information of the subject from a user terminal;
analyzing the acquired medical data; and
transmitting an analysis result and the second identification information to the user terminal.

14. An analysis program for causing a computer to function as the analysis device according to any one of claims 1 to 12, and for causing a computer to function as the acquiring unit, the analyzing unit, and the transmitter.

15. A computer-readable non-transitory recording medium in which the analysis program according to claim 14 has been recorded.
